# EUROPÄISCHE PATENTSCHRIFT

(11) **EP 0 892 777 B1**
(45) Veröffentlichungstag und Bekanntmachung des Hinweises auf die Patenterteilung: **13.09.2000**
(21) Anmeldenummer: 97918103.9
(22) Anmeldetag: 03.04.1997
(51) Int. Cl.: C07C 209/48, B01J 38/10

(54) **VERFAHREN ZUR HERSTELLUNG VON AMINEN UND AMINONITRILEN**
METHOD OF PRODUCING AMINES AND AMINONITRILES
PROCEDE DE FABRICATION D'AMINES ET D'AMINONITRILES

(30) Priorität: 11.04.1996 DE 19614283
(43) Veröffentlichungstag der Anmeldung: 27.01.1999
(73) Patentinhaber: BASF AKTIENGESELLSCHAFT, 67056 Ludwigshafen (DE)
(72) Erfinder: SCHNURR, Werner, D-67273 Herxheim (DE); VOIT, Guido, D-69198 Schriesheim (DE); FLICK, Klemens, D-76863 Herxheim (DE); FISCHER, Rolf-Hartmuth, D-69121 Heidelberg (DE)
(86) Internationale Anmeldenummer: EP9701672
(87) Internationale Veröffentlichungsnummer: WO9737963

(56) Entgegenhaltungen:
- GB-A- 728 599
- US-A- 3 056 837
- US-A- 3 758 584

## Beschreibung

Die vorliegende Erfindung betrifft ein verbessertes Verfahren zur Herstellung von NH₂-Gruppen enthaltenden Verbindungen durch Hydrierung von Verbindungen, die mindestens eine ungesättigte Kohlenstoff-Stickstoff-Bindung enthalten, mit Wasserstoff in Gegenwart eines Katalysators bei Temperaturen von nicht unterhalb Raumtemperatur und erhöhtem Wasserstoff-Partialdruck gewünschtenfalls in Gegenwart eines Lösungsmittels.

Des weiteren betrifft die vorliegende Erfindung ein Verfahren zur Herstellung von insbesondere 6-Aminocapronitril ("ACN") und Hexamethylendiamin ("HMD"), ein Verfahren, indem man die Hydrierung von mindestens eine ungesättigte Kohlenstoff-Stickstoff-Bindung enthaltenden Verbindungen in Suspension oder in einem Festbettreaktor in Riesel- oder Sumpffahrweise durchführt sowie ein Verfahren zur Regenerierung von Cobalt- und/oder Eisen-haltigen Katalysatoren.

Die Hydrierung von ungesättigten Kohlenstoff-Stickstoff-Bindungen mit Wasserstoff ist beispielsweise in Houben-Weyl, Bd. 11/1 (Stickstoff-Verbindungen II, Amine), S. 545-574, 4.Auflage, 1957, beschrieben.

Aus der US-A 2,257,814 ist ein Verfahren zur Herstellung von Aminonitrilen aus Dinitrilen bekannt, wobei man die Hydrierung in flüssiger Phase in Gegenwart von Cobalt- und gewünschtenfalls Eisen-haltigen Katalysatoren durchführt. Des weiteren beschreibt die DE-A 848,654 die partielle Hydrierung von Adipodinitril ("ADN") zu ACN in Gegenwart von Festbettkatalysatoren auf der Basis von Kupfer/Cobalt/Zink- und Eisen-Cobalt-Spinellen. Die DE-A 954,416 beschreibt die Verwendung von Cobalt auf Kieselgel als Katalysator zur Herstellung von Aminonitrilen und Diaminen durch Hydrierung von Dinitrilen mit Wasserstoff. Die DE-A 4,235,466 beschreibt ein Verfahren zur Herstellung von cycloaliphatischen und aliphatischen Aminonitrilen durch katalytische Hydrierung in Gegenwart eines Katalysators, der aus Eisenschwamm hergestellt wird.

Die bei der Hydrierung von Nitrilen und Iminen eingesetzten Cobalt- und Eisen-haltigen Katalysatoren verlieren in Langzeitversuchen an Aktivität und müssen durch neue Katalysatoren ersetzt werden, wenn gewisse Grenzwerte hinsichtlich Umsatz und/ oder Selektivität oder Nebenproduktmenge erreicht sind.

Die Regenerierung von mit Kohlenstoff-haltigen Ablagerungen belegten Katalysatoren erfolgt in der Regel durch Abbrennen der organischen Beläge mit Stickstoff/Luft-Gemischen (Chem. Eng. Sci., Vol.46 (1991) S. 11-21). Diese Methode kann jedoch nur bei solchen Katalysatoren angewandt werden, die bei der Umsetzung mit Luft mechanisch stabil bleiben. Bei Trägerkatalysatoren, die ein stabiles Gerüst aus oxidischem Material, wie SiO₂, Al₂O₃, TiO₂ aufweisen, kann diese Regenerierungsmethode mit Erfolg eingesetzt werden. So wird in GB-A 2,284,163 die Regenerierung eines Trägerkatalysators mit Pt, Pd, Ru, Rh, Os, Ir oder Ni durch eine Behandlung eines mindestens Chlor und Sauerstoff enthaltenden Gases beschrieben.

Katalysatoren mit sehr hohen Metallgehalten nehmen durch Abbrennen der organischen Beläge durch Luft Schaden und verändern ihre mechanischen Eigenschaften (siehe beispielsweise EP-A 61,042).

Es ist aus Journal of Catalysis 143 (1993) S. 187-200 bekannt, daß man einen Nickel-Katalysator (25 Gew.-% Ni auf SiO₂), der für die Hydrierung von Acetonitril in der Gasphase eingesetzt wird, durch Behandlung mit Wasserstoff bei Temperaturen über 200°C regenerieren kann.

Aus dem genannten Stand der Technik ist nicht zu entnehmen, ob sich unter diesen Bedingungen auch Cobalt- und/oder Eisen-haltige Katalysatoren regenerieren lassen.

Der vorliegenden Erfindung lag daher die Aufgabe zugrunde, ein Verfahren zur Verfügung zu stellen, das es gestattet, die bei der Hydrierung einer mindestens eine ungesättigte Kohlenstoff-Stickstoff-Bindung enthaltende Verbindung eingesetzten Cobalt- und/ oder Eisen-haltigen Katalysatoren mit einfachen Mitteln zu regenerieren, so daß es zu keinen langen Stillstandzeiten während der Regenerierung der Katalysatoren kommt. Insbesondere war es das Ziel, die Katalysatoraktivität hinsichtlich Umsatz und Selektivität bei der Hydrierung der mindestens eine ungesättigte Kohlenstoff-Stickstoff-Bindung enthaltenden Verbindung möglichst wieder auf das Niveau des ungebrauchten Katalysators anzuheben.

Demgemäß wurde ein Verfahren zur Herstellung von NH₂-Gruppen enthaltenden Verbindungen durch Hydrierung von Verbindungen, die mindestens eine ungesättigte Kohlenstoff-Stickstoff-Bindung enthalten, mit Wasserstoff in Gegenwart eines Katalysators bei Temperaturen von nicht unterhalb Raumtemperatur und erhöhtem Wasserstoff-Partialdruck gewünschtenfalls in Gegenwart eines Lösungsmittels gefunden, indem man
(a) als Katalysator einen Cobalt- und/oder Eisen-haltigen Katalysator einsetzt, und
(b) nach Absinken des Umsatzes, bezogen auf die zu hydrierende Verbindung, und/oder der Selektivität, bezogen auf das gewünschte Produkt, unter einen definierten Wert oder nach Ansteigen der Menge eines unerwünschten Nebenproduktes über einen definierten Wert, die Hydrierung unterbricht, indem man die Zufuhr der zu hydrierenden Verbindungen und gewünschtenfalls vorhandenem Lösungsmittel stoppt,
(c) den Katalysator bei einer Temperatur im Bereich von 150 bis 400°C mit Wasserstoff behandelt, wobei man den Wasserstoffdruck im Bereich von 0,1 bis 30 MPa und die Behandlungszeit im Bereich von 2 bis 48 h wählt, und
(d) anschließend die Hydrierung der mindestens eine ungesättigte Kohlenstoff-Stickstoff-Bindung enthaltende Verbindungen fortsetzt.

Des weiteren wurden ein Verfahren, indem man mindestens eine ungesättigte Kohlenstoff-Stickstoff-Bindung enthaltende Verbindungen in Suspension oder in einem Festbettreaktor in Riesel- oder Sumpffahrweise hydriert, ein Verfahren zur Herstellung von insbesondere 6-Aminocapronitril ("ACN") und Hexamethylendiamin ("HMD") sowie ein Verfahren zur Regenerierung von Cobalt-und Eisen-haltigen Katalysatoren gefunden.

Als Ausgangsverbindungen setzt man erfindungsgemäß Verbindungen, die mindestens eine ungesättigte Kohlenstoff-Stickstoff-Bindung enthalten, wie eine Kohlenstoff-Stickstoff-Doppel- oder Dreifachbindung ein. Bevorzugt setzt man ein C₄-C₈-Alkylnitrile oder -dinitrile wie Butannitril, Pentannitril, Hexannitril, Heptannitril, Oktannitril, Butandinitril ("Adipodinitril", kurz "ADN"), Pentandinitril, Hexandinitril, Heptandinitril und Oktandinitril, insbesondere Adipodinitril, besonders bevorzugt endständige C₄-C₈-Dinitrile wie 1,4-Dicyanbutan (Adipodinitril), 1,5-Dicyanpentan, 1,6-Dicyanhexan, 1,7-Dicyanheptan und 1,8-Dicyanoctan, insbesondere Adipodinitril, C₅-C₈-Cycloalkylnitrile oder -dinitrile wie Cyclopentylnitril, Cyclohexylnitril, Cycloheptylnitril, Cyclooctylnitril, Cyclopentyldinitril, Cyclohexyldinitril, sowie 4 bis 8 Kohlenstoffatome enthaltende Aminonitrile, bevorzugt α,ω-Aminonitrile wie 5-Aminovaleronitril und 6-Aminocapronitril ("ACN"), insbesondere ACN.

Die Nitrile, Dinitrile und Aminonitrile können auch andere funktionelle Gruppen tragen soweit diese die Hydrierung nicht beeinträchtigen, oder deren gleichzeitige oder teilweise Hydrierung gewünscht ist. Beispielhaft seien C₁-C₄-Alkylgruppen, Arylgruppen, insbesondere Phenyl, C₅-C₈-Cycloalkyl, Aminoalkyl, N-Alkyl-aminoalkyl, N-(Cyanomethyl)-aminoalkyl und die Iminogruppe (C=NH, C=NR) genannt, vorzugsweise die Iminogruppe.

Besonders bevorzugte Verbindungen sind ADN, ACN, 3-Cyano-3,5,5-trimethylcyclohexylimin, NC-(CH₂)₂-N(H)-(CH₂)₂-CN, NC-(CH₂)₂-N(H)-(CH₂)₂-N(H)-(CH₂)₂-CN und 1-Cyano-2-amino-ethan.

Die Cobalt- und/oder Eisen-Katalysatoren kann man ohne Träger, insbesondere für Festbett- oder Suspensionsfahrweise, beispielsweise in Form von Raney-Katalysatoren oder in anderen üblichen ungeträgerten Formen (sogenannte Vollkontakt-Katalysatoren) einsetzen. Die ungeträgerten Formen können im Vergleich zum hohen Gehalt der aktiven Komponenten geringe Beimengungen enthalten. Diese Beimengungen können günstige Auswirkungen auf entweder die katalytische Aktivität und/oder Selektivität, oder auch auf Eigenschaften wie Härte, Abrieb, chemische oder thermische Beständigkeit des Katalysators haben. Die Gesamtmenge der Beimengungen liegt im allgemeinen im Bereich von 0 bis 20 Gew.-%, bezogen auf die Menge der aktiven Komponente. Als Beimengungen kann man Oxide, Phosphate und Sulfate der Alkalimetall- und Erdalkalimetallverbindungen, thermisch stabile Oxide wie SiO₂, Al₂O₃, TiO₂ und ZrO₂ sowie andere Übergangsmetalloxide einsetzen. Ein Einsatz in Form eines Trägerkatalysators ist ebenfalls möglich. Als Träger kann man üblicherweise Aluminiumoxid, Siliciumdioxid, Aktivkohlen, Titandioxid und Zirkondioxid verwenden. Bei geträgerten Katalysatoren liegt der Gehalt an Cobalt und/oder Eisen, je nachdem, ob nur eines dieser Elemente oder beide vorhanden sind, zu Träger in der Regel im Bereich von 3 bis 95, bevorzugt von 30 bis 95 Gew.-%.

Die Katalysatoren kann man auch, wenn gewünscht, mit Metallen der Gruppe VIB (Cr, Mo, W), VIII des Periodensystems der Elemente (Ru, Os, Rh, Ir, Pd, Pt) sowie Kupfer, Mangan und Rhenium modifizieren, wobei der Cobalt- bzw. Eisen-Gehalt im Katalysator im allgemeinen im Bereich von 50 bis 99,9, bevorzugt von 80 bis 99 Gew.-%, bezogen auf die aktiven Komponenten (Cobalt und/oder Eisen + Modifizierungsmittel), beträgt.

Des weiteren kann man die Katalysatoren mit einer Verbindung auf der Basis eines Alkalimetalles oder Erdalkalimetalles wie Lithium, Natrium, Kalium, Rubidium, Cäsium, Magnesium, Calcium, Strontium und Barium, insbesondere Cäsium, modifizieren. Üblicherweise wählt man ein Gewichtsverhältnis im Bereich von 0 bis 5, bevorzugt von 0,1 bis 3 Gew.-% Alkalimetall bzw. Erdalkalimetall, bezogen auf Masse an Cobalt und Eisen (wobei eines der Elemente Co und Fe nicht vorhanden sein muß).

Bevorzugte Katalysatoren sind Eisen- und Cobalt-Katalysatoren ohne Träger (sogenannte Vollkontaktkatalysatoren) mit einem Gehalt an Eisen und/oder Cobalt von mindestens 60 Gew.-%, bezogen auf die Masse an Cobalt und/oder Eisen sowie gewünschtenfalls Modifizierungsmittel, soweit vorhanden.

Zur Herstellung von Fe-Katalysatoren, die hauptsächlich Verwendung finden in der Ammoniak-Synthese, der Fischer-Tropsch-Reaktion oder als Dehydrierkatalysator zur Herstellung von Styrol aus Ethylbenzol, sind in der Literatur verschiedene Wege beschrieben. So können Fe-Katalysatoren aus natürlich vorhandenen Fe-Oxiden wie Hämatit oder Magnetit oder künstlich durch Oxidation von metallurgisch erzeugtem Eisen hergestellt werden (siehe Ullmann's Encyclopedia of Industrial Chemistry, 5th Ed., Vol A2, S. 169-172). Modifizierungsmittel (oder auch Promotoren genannt) werden üblicherweise durch gemeinsames Aufschmelzen der Oxide eingebracht oder durch nachträgliche Imprägnierung der Fe-Oxide auf die innere Oberfläche aufgebracht. Die Eisenoxid-Precursor können aber auch durch Fällung (siehe z.B. B.E. Leach, Applied Industrial Catalysis, Vol. 2, 1983, S. 177- 180) oder Mit- oder Co-Fällung auf inerte oxidische Materialien aus wäßrigen Eisensalz-Lösungen als Carbonate oder Hydroxide erhalten werden. Diese Precursor kann man in an sich bekannter Weise durch Tablettieren oder Verstrangen in eine technisch einsetzbare Form bringen (A.B. Stiles, Catalyst manufacture, New York 1983, S. 137-138, oder M. Sittig, Catalyst Manufacture, Recovery and Use, 1972, Noyes data corporation, S.217-221).

Eine weitere Möglichkeit zur Herstellung von Fe-Katalysatoren ist beispielsweise die thermische Zersetzung von Fe-Cyaniden zu Fe-Carbiden und Fe-Nitriden, die in der Regel bei weiterem Erhitzen in alpha-Eisen überführt werden können (siehe Ullmann's Encyclopedia of Industrial Chemistry, 5th Ed., Vol A2, S. 169-172).

Cobalt-Katalysatoren können durch Imprägnierung eines keramischen Trägers mit wäßrigen oder organischen Lösungen einer Cobalt-haltigen Verbindung hergestellt werden. Die Imprägnierung kann am fertigen Trägerstrang oder aber auf dem Trägerpulver durchgeführt werden. Falls man den keramischen Träger als Pulver einsetzt, dann verformt man üblicherweise das mit Cobalt imprägnierte Pulver durch beispielsweise Verstrangen oder Tablettieren, bevorzugt nach Calcinierung.

Für den Fall, daß man nach einem Imprägnierungsschritt nicht genügend Cobalt auf den Träger aufgebracht hat, beispielsweise bedingt durch die Löslichkeit der eingesetzten Cobaltsalze oder der Oberfläche des Trägers, so kann man nach den bisherigen Beobachtungen den Imprägnierungsvorgang so oft wiederholen, bis die gewünschte Menge an Cobalt aufgebracht ist, wobei man nach jedem Imprägnierungsschritt die erhaltene Masse trocknet und calciniert, bevor man den nächsten Imprägnierungsvorgang durchführt.

Es ist auch möglich, Cobalt-haltige Katalysatoren durch eine Fällung aus wäßriger oder organischer Lösung herzustellen, wobei die Modifizierungsmittel (oder Promotoren) üblicherweise mitgefällt oder nachfolgend durch Imprägnierung aufgebracht werden können. Man fällt bevorzugt das Cobalthydroxid oder das entsprechende Carbonat oder andere schwerlösliche Cobaltverbindungen aus. Nach der Fällung trocknet man in der Regel den ausgefällten Niederschlag, verarbeitet dann im allgemeinen die getrocknete Masse durch beispielsweise Verstrangen oder Tablettieren, wobei man gegebenenfalls vor der Verformung zu Strängen oder Tabletten eine Calcinierung bei Temperaturen im Bereich von 200 bis 700°C durchführen kann, um bestimmte, gewünschte Festkörperphasen zu erhalten.

Vor dem Einsatz als Hydrierkatalysatoren werden die Cobaltoxid-und/oder Eisenoxid-haltigen Vorkatalysatoren zweckmäßig durch eine Wasserstoffbehandlung zu den entsprechenden Metallen reduziert, wobei in der Regel ein Oxidgehalt von nicht größer als 10 Gew.-%, bevorzugt von nicht größer als 5 Gew.-%, besonders bevorzugt von nicht größer als 1 Gew.-%, bezogen auf die Gesamtmasse von Metall und Oxid, nach bisherigen Erkenntnissen bevorzugt ist. Diese Reduzierung der oxidhaltigen Massen zu den entsprechenden aktiven Katalysatormassen kann man drucklos oder bei erhöhtem Druck bei Temperaturen ab 200°C in an sich bekannter Art und Weise durchführen.

Die Hydrierungen kann man in Sumpf-, Riesel- oder Suspensionsfahrweise durchführen.

Führt man die Umsetzung in einer Suspension durch, wählt man üblicherweise Temperaturen im Bereich von 40 bis 150, vorzugsweise von 50 bis 100, besonders vorzugsweise von 60 bis 90°C; den Druck wählt man im allgemeinen im Bereich von 2 bis 20, vorzugsweise von 3 bis 10, besonders bevorzugt von 4 bis 9 MPa. Die Verweilzeiten sind im wesentlichen von der gewünschten Ausbeute, Selektivität und dem gewünschten Umsatz abhängig; üblicherweise wählt man die Verweilzeit so, daß ein Maximum an Ausbeute erreicht wird, beispielsweise im Bereich von 50 bis 275, vorzugsweise von 70 bis 200 min.

Bei der Suspensionsfahrweise kann man als Lösungsmittel bevorzugt Ammoniak, Amine, Diamine und Triamine mit 1 bis 6 C-Atomen wie Trimethylamin, Triethylamin, Tripropylamin und Tributylamin oder Alkohole, insbesondere Methanol und Ethanol, besonders bevorzugt Ammoniak einsetzen. Zweckmäßig wählt man eine Konzentration an zu hydrierender Verbindung im Bereich von 10 bis 90, vorzugsweise von 30 bis 80, besonders vorzugsweise von 40 bis 70 Gew.-%, bezogen auf die Summe von zu hydrierender Verbindung und Lösungsmittel.

Die Menge an Katalysator wählt man im allgemeinen so, daß die Katalysator-Menge im Bereich von 1 bis 50, bevorzugt von 5 bis 20 Gew.-%, bezogen auf die eingesetzte Menge an zu hydrierender Verbindung, beträgt.

Die Suspensionshydrierung kann man diskontinuierlich oder, bevorzugt kontinuierlich, in der Regel in der Flüssigphase durchführen.

Man kann die Hydrierung auch diskontinuierlich oder kontinuierlich in einem Festbettreaktor in Riesel- oder Sumpffahrweise durchführen, wobei man üblicherweise eine Temperatur im Bereich von 30 bis 200, vorzugsweise von 50 bis 150°C und einen Druck in der Regel im Bereich von 2 bis 30, vorzugsweise von 3 bis 20 MPa wählt. Bevorzugt führt man die Hydrierung in Gegenwart eines Lösungsmittels, bevorzugt Ammoniak, Amine, Diamine und Triamine mit 1 bis 6 C-Atomen wie Trimethylamin, Triethylamin, Tripropylamin und Tributylamin oder Alkohol, bevorzugt Methanol und Ethanol, besonders bevorzugt Ammoniak durch. In einer bevorzugten Ausführungsform wählt man einen Gehalt an Ammoniak im Bereich von 0,5 bis 10, bevorzugt von 1 bis 6 g pro zu hydrierender Verbindung, insbesondere Adipodinitril. Bevorzugt wählt man dabei eine Katalysatorbelastung im Bereich von 0,1 bis 2,0, vorzugsweise von 0,3 bis 1,0 kg der zu hydrierenden Verbindung/l.h, insbesondere Adipodinitril/l*h. Auch hier kann man durch Veränderung der Verweilzeit den Umsatz und damit die Selektivität gezielt einstellen.

Die Hydrierung kann man in einem üblichen hierfür geeigneten Reaktor durchführen.

Führt man die Umsetzung in der Gasphase durch, wählt man üblicherweise Temperaturen im Bereich von 100 bis 250, vorzugsweise von 160 bis 200°C; den Druck wählt man im allgemeinen im Bereich von 0,01 bis 3, vorzugsweise von 0,09 bis 0,5 MPa. Des weiteren setzt man in der Regel 2 bis bis 300, bevorzugt von 10 bis 200 Mol Wasserstoff pro Mol Verbindung, die mindestens eine ungesättigte Kohlenstoff-Stickstoff-Bindung enthält, ein.

In einer bevorzugten Ausführungsform führt man die Hydrierung mit ADN in Gegenwart von Ammoniak als Lösungsmittel mit Festbettkatalysatoren durch, wobei man nach Desaktivierung des Katalysators, d. h. Absinken von Umsatz, bezogen auf ADN, und/oder Selektivität, bezogen auf ACN, unter einen definierten Wert, zunächst den Zulauf an Adipodinitril und Ammoniak abgestellt, anschließend die Temperatur auf 200 bis 250°C bringt und dann den Katalysator zehn bis zwanzig Stunden mit von 200 bis 800, bevorzugt von 500 bis 700, insbesondere 600 1 Wasserstoff / l Kat. x h behandelt. Danach kühlt man üblicherweise auf Reaktionstemperatur ab und setzt die Hydrierung fort.

Vor Beginn der Regenerierung entfernt man bevorzugt den noch im 5 Reaktor enthaltenen Hydrieraustrag. Es kann vorteilhaft sein, den Katalysator vor der eigentlichen Regenerierung mit dem im System enthaltenen Lösungsmittel, insbesondere Ammoniak, zu waschen. Die Temperatur beim Waschen wählt man üblicherweise im Bereich von 20 bis 200°C, insbesondere von 20 bis 100°C. Für das Waschen ist in der Regel ein Zeitraum von 2 bis 24 Stunden vorteilhaft.

Die Regenerierung der Katalysatoren führt man erfindungsgemäß bei Temperaturen im Bereich von 150 bis 400°C, vorzugsweise 180 bis 350°C, insbesondere von 200 bis 300°C durch, wobei man den Wasserstoffdruck im Bereich von 0,1 bis 30 MPa, bevorzugt 0,1 bis 20 MPa wählt. Bei kontinuierlicher Fahrweise wählt man die Wasserstoffmenge üblicherweise im Bereich von 100 bis 1500, vorzugsweise von 200 bis 1000 l Wasserstoff/ l Reaktorvolumen x Stunde.

Mit Hilfe des erfindungsgemäßen Verfahrens ist es möglich, die Katalysatorstandzeit und die Raum-Zeit-Ausbeute von Cobalt- und/ oder Eisen-haltigen Katalysatoren bei der Hydrierung von Verbindungen, die mindestens eine ungesättigte Kohlenstoff-Stickstoff-Bindung enthalten, insbesondere bei der Hydrierung von Adipodinitril zu Aminocapronitril und Hexamethylendiamin (Zwischenprodukte bei der Nylon 6- und Nylon 66-Herstellung), deutlich zu verbessern.

### Beispiele

### Beispiel 1 - Herstellung eines Cobalt-haltigen Vollkatalysators

Zu einer wäßrigen Lösung aus Kobaltnitrat, Mangannitrat und Phosphorsäure in Wasser, die 10 Gew.-% Cobalt (der Gehalt an Cobalt wurde ausgehend von Cobaltnitrat berechnet), 0,55 Gew.-% Mangan (berechnet aus der Menge an Mangannitrat) und 0,45 Gew.-% H₃PO₄ enthielt, wurde bei einer Temperatur von 50°C eine 20 gew.-%ige Natriumcarbonatlösung portionsweise so zugegeben, daß sich nach Zugabe der Natriumcarbonatlösung stets ein pH-Wert von 6 einstellte, wobei die entsprechenden Carbonate ausgefällt wurden. Nach beendeter Fällung, die daran erkennbar war, daß sich der eingestellte pH-Wert von 6 nicht mehr änderte, wurde weitere Natriumcarbonatlösung solange zugegeben, bis sich ein pH-Wert von 7,5 einstellte. Der entstandene Niederschlag wurde "Nitrat- und Natrium-frei" gewaschen, indem man den Niederschlag solange mit Wasser wusch, bis eine End-Leitfähigkeit von 20 µSiemens erreicht war und mittels Merckoquant®-Teststäbchen für Nitrat (Fa.Merck) der Nitratgehalt in der Lösung kleiner als 0,02 Gew.-% betrug. Der so gewaschene Niederschlag wurde mit Wasser angemaischt und in einem Sprühturm versprüht (Eingangstemperatur = 550°C). Das Sprühgut wurde bei 500°C getrocknet, gekollert und im Extruder zu Strängen von 4 mm Durchmesser und 1 cm Länge verformt. Die Stränge wurden bei 100 bis 120°C getrocknet und 1 h bei 900°C calziniert. Das calcinierte Produkt setzte sich aus 90 Gew.-% CoO, 5 Gew.-% Mn₂O₃, 3 Gew.-% P₂O₅ und 2 Gew.-% Na₂O zusammen. Die so erhaltenen Stränge wurden bei 320°C im Wasserstoffstrom während 16 h reduziert und bei Raumtemperatur mit einem Stickstoff/Luft-Gemisch (5 Vol.-% Luft, 95 Vol.-% Stickstoff) passiviert.

### Beispiel 2 - Herstellung eines Eisen-haltigen Vollkatalysators

Nach der in Catalyst Manufacture, A.B.Stiles, T.A.Koch (1995) S. 167/68 beschriebenen Methode wurde eine Mischung aus Fe-Oxid (Magnetit) mit den Promotoren Al₂O₃, K₂CO₃ und Ca-Carbonat bei einer Temperatur von 1600 bis 2000°C aufgeschmolzen. Anschließend wurde die Schmelze abgekühlt und zerkleinert. Die erhaltene Masse (Katalysator im oxidischen Zustand) hatte folgende Zusammensetzung: 1,1 Gew.-% K₂O, 3,0 Gew.-% Al₂O₃, 2,3 Gew.-% CaO, der Rest bestand aus FeO und Fe₂O₃. Um einen einsatzfähigen Katalysator zu erhalten, wurde die erhaltene Masse bei einem Druck von 3 MPa mit Wasserstoff bei einer Temperatur von 450°C behandelt während 32 h und anschließend bei Raumtemperatur mit einem Stickstoff/Luft-Gemisch (5 Vol.-% Luft, 95 Vol.-% Stickstoff) passiviert. Das Verhältnis von Masse an Metallen zu Masse an Oxiden ("Reduktionsgrad") betrug 9:1.

### Beispiel 3 - Festbetthydrierung in der Flüssigphase

Ein Rohrreaktor von 2 m Länge und 2,5 cm Innendurchmesser wurde mit 750 ml (1534 g) des passivierten Katalysators aus Beispiel 1 befüllt. Dann wurde der passivierte Katalysator innerhalb von 48 h in einem Wasserstoffstrom (500 1/h) durch Erhöhung der Temperatur von 30°C auf 280°C drucklos (d.h. bei Atmosphärendruck) aktiviert (in dieser Zeit wurde noch nicht reduziertes CoO zu Co reduziert).

Nach Absenkung der Temperatur auf 45°C (gemessen am Eingang des Reaktors) bzw. 85°C (gemessen am Ausgang des Reaktors) wurde dem Reaktor bei 20 MPa Gesamtdruck ein Gemisch aus 400 ml/h Adipodinitril, 600 ml/h Ammoniak und 500 l/h Wasserstoff zugeführt. Zusätzlich wurde zur Wärmeabfuhr etwa die vierfache Zulaufmenge (4,2 l/h) im Kreis gefahren, wobei der Kreisstrom über einen Wärmetauscher geführt wurde. Das Adipodinitril setzte sich unter diesen Bedingungen zu 70 % um. Das Reaktionsgemisch enthielt zu Beginn 30 Gew.-% ADN, 35 Gew.-% ACN und 34,5 Gew.-% HMD (ACN-Selektivität: 50 %, ACN + HMD-Selektivität: > 99 %). Nach einer Versuchsdauer von 3600 h fiel die ACN-Selektivität, bei unverändertem Umsatz von anfänglich 50 auf 23%.

Anschließend wurde der Dinitril- sowie der Ammoniak-Zulauf abgestellt und der Katalysator im Reaktor 12 Stunden bei 200°C und einem Gesamtdruck von 200 bar (bei 500 l/h Wasserstoff) regeneriert. Nach erneutem Anfahren unter gleichen Bedingungen (s. o.) wurde ein Selektivitätsanstieg auf 50 % erhalten, d. h. die Anfangsselektivität des Katalysators wurde wieder erreicht.

### Beispiel 4 - Festbetthydrierung in der Flüssigphase

Ein Rohrreaktor von 2 m Länge und 2,5 cm Innendurchmesser wurde mit 800 ml (1598 g) des passivierten Katalysators aus Beispiel 1 befüllt. Der Katalysator wurde dann innerhalb von 48 h in einem Wasserstoffstrom (500 l/h) durch Erhöhung der Temperatur von 30°C auf 320°C drucklos (d.h. bei Atmosphärendruck) aktiviert (in dieser Zeit wurde restliches CoO zu Co reduziert).

Nach Absenkung der Temperatur auf 120°C (gemessen am Eingang des Reaktorrohres) bzw. 140°C (gemessen am Ausgang des Reaktorrohres) wurde dem Reaktor in Rieselfahrweise bei 25 MPa ein Gemisch aus 180 ml/h 3-Cyano-3,5,5-trimethylcyclohexylimin, 1700 ml/h Ammoniak und 500 ml/h Wasserstoff zugeführt. Das Imin setzte sich unter diesen Bedingungen zu 100 % um. Die Ausbeute an 3-Aminomethyl-3,5,5-trimethylcyclohexylamin betrug 94 % (Selektivität: 94 %). Zwischenprodukt der Hydrierung und damit Indikator für die Katalysatoraktivität war 3-Cyano-3,5,5-trimethylcyclohexylamin. Die Konzentration an dieser Verbindung stieg von anfänglich 0 auf 1500 ppm (bezogen auf das Reaktionsgemisch) nach 5700 h an, so daß eine Regenerierung des Katalysators aus Gründen der Produktspezifikation erforderlich erschien.

Anschließend wurde der Nitril- sowie der Ammoniak-Zulauf abgestellt und der Katalysator im Reaktor 24 h bei 300°C und einem Gesamtdruck von 25 MPa (bei 500 l/h Wasserstoff) regeneriert. Nach erneutem Anfahren unter gleichen Bedingungen wie oben, wurde ein Abfall der Zwischenprodukt-Konzentration auf 200 ppm erhalten, d.h. die Anfangsaktivität des Katalysators wurde nahezu erreicht.

### Beispiel 5 - Festbetthydrierung in der Flüssigphase

Ein Rohrreaktor von 7 m Länge und 10,5 cm Innendurchmesser wurde mit 60 l (130 kg) des in Beispiel 2 erhaltenen Katalysators (Reduktionsgrad 9:1) befüllt und anschließend wurde der Katalysator bei einer Temperatur von 370°C und einem Gesamtdruck von 15 MPa während 72 h aktiviert (hierbei wurde das verbliebene Eisenoxid zu Eisen reduziert), indem man zunächst Stickstoff durch den Reaktor leitete, und den Stickstoff dann stufenweise während der ersten 24 h durch Wasserstoff ersetzte.

Nach Absenken der Temperatur auf 110°C (gemessen am Eingang des Reaktors) bzw. 135°C (gemessen am Ausgang des Reaktors), wurde dem Reaktor bei einem Gesamtdruck von 25 MPa ein Gemisch aus 30 kg/h ADN, 50 l/h fl.Ammoniak und 40 Nm³/h Wasserstoff zugeführt. Zusätzlich wurde zur Wärmeabfuhr die fünffache Zulaufmenge (400 l/h) im Kreis gefahren (Abführung der Wärme erfolgte über einen Wärmetauscher, so daß am Eingang des Reaktors eine Temperatur des Kreisstromes von 110°C erreicht wurde). Das ADN setzte sich unter diesen Bedingungen zu 70 % um. Das Reaktionsgemisch enthielt zu Beginn 30 Gew.-% ADN, 35 Gew.-% ACN und 34,5 Gew.-% HMD (ACN-Selektivität: 50 %, ACN- + HMD-Selektivität: > 99%). Nach 800 Stunden wurde der Katalysator durch Abstellen der Zuläufe ohne Spülung gezielt desaktiviert.

Zur Regenerierung wurde der Katalysator im Reaktor zunächst mit Stickstoff (80 m3/h, bei einer Temperatur im Bereich von 200 bis 250°C, und einem Druck von 15 MPa während 24 h) behandelt. Anschließend wurde auf 270°C erhitzt und während 5 h Stickstoff (80 m³/h) schrittweise durch Wasserstoff ersetzt. Während des Austauschvorganges von Stickstoff durch Wasserstoff wurde auch die Temperatur schrittweise auf 380°C erhöht. Abschließend wurde der Reaktor 24 h bei einer Temperatur im Bereich von 350 bis 380°C und einem Wasserstoffdruck von 20 MPa gehalten. Nach erneutem Anfahren unter den gleichen Bedingungen wie oben angegeben, wurde die Anfangsselektivität des Katalysators wieder erreicht.

## Patentansprüche

1. Verfahren zur Herstellung von NH₂-Gruppen enthaltenden Verbindungen durch Hydrierung von Verbindungen, die mindestens eine ungesättigte Kohlenstoff-Stickstoff-Bindung enthalten, mit Wasserstoff in Gegenwart eines Katalysators bei Temperaturen von nicht unterhalb Raumtemperatur und erhöhtem Wasserstoff-Partialdruck gewünschtenfalls in Gegenwart eines Lösungsmittels, dadurch gekennzeichnet, daß man
(a) als Katalysator einen Cobalt- und/oder Eisen-haltigen Katalysator einsetzt, und
(b) nach Absinken des Umsatzes, bezogen auf die zu hydrierende Verbindung, und/oder der Selektivität, bezogen auf das gewünschte Produkt, unter einen definierten Wert oder nach Ansteigen der Menge eines unerwünschten Nebenproduktes über einen definierten Wert, die Hydrierung unterbricht, indem man die Zufuhr der zu hydrierenden Verbindungen sowie gewünschtenfalls vorhandenem Lösungsmittel stoppt,
(c) den Katalysator bei einer Temperatur im Bereich von 150 bis 400°C mit Wasserstoff behandelt, wobei man den Wasserstoffdruck im Bereich von 0,1 bis 30 MPa und die Behandlungszeit im Bereich von 2 bis 48h wählt, und
(d) anschließend die Hydrierung der mindestens eine ungesättigte Kohlenstoff-Stickstoff-Bindung enthaltenden Verbindungen fortsetzt.

2. Verfahren nach Anspruch 1, dadurch gekennzeichnet, daß man als mindestens eine ungesättigte Kohlenstoff-Stickstoff-Bindung enthaltende Verbindungen C₄-C₈-Alkylnitrile, C₅-C₈-Cycloalkylnitrile, C₄-C₈-Alkyldinitrile oder C₅-C₈-Cycloalkyldinitrile einsetzt.

3. Verfahren nach Anspruch 1 oder 2, dadurch gekennzeichnet, daß man Adipodinitril unter Erhalt von 6-Aminocapronitril und Hexamethylendiamin einsetzt.

4. Verfahren gemäß den Ansprüchen 1 bis 3, dadurch gekennzeichnet, daß man die Hydrierung der mindestens eine ungesättigte Kohlenstoff-Stickstoff-Bindung enthaltenden Verbindung in Suspension bei einer Temperatur im Bereich von 40 bis 150°C und einem Druck im Bereich von 2 bis 20 MPa durchführt.

5. Verfahren gemäß den Ansprüchen 1 bis 3, dadurch gekennzeichnet, daß man die Hydrierung der mindestens eine ungesättigte Kohlenstoff-Stickstoff-Bindung enthaltenden Verbindung in einem Festbettreaktor in Riesel- oder Sumpffahrweise bei einer Temperatur im Bereich von 30 bis 200°C und einem Druck im Bereich von 2 bis 30 MPa durchführt.

6. Verfahren zur Regenerierung von Cobalt- und/oder Eisen-haltigen Katalysatoren, dadurch gekennzeichnet, daß man die Katalysatoren mit Wasserstoff bei einer Temperatur im Bereich von 150 bis 400°C und einem Wasserstoffdruck im Bereich von 0,1 bis 30 MPa und einer Behandlungszeit im Bereich von 2 bis 48 h behandelt.

## Claims

1. A process for preparing an NH₂-containing compound by hydrogenating a compound containing at least one unsaturated carbon-nitrogen bond with hydrogen in the presence of a catalyst at temperatures not below room temperature and elevated hydrogen partial pressure in the presence or absence of a solvent, which comprises
(a) using a catalyst comprising a cobalt- and/or iron-containing catalyst, and
(b) after the conversion based on the compound to be hydrogenated and/or the selectivity based on the desired product has or have dropped below a defined value or the amount of an unwanted by-product has risen beyond a defined value, interrupting the hydrogenation by stopping the feed of the compound to be hydrogenated and of the solvent, if used,
(c) treating the catalyst at from 150 to 400°C with hydrogen using a hydrogen pressure within the range from 0.1 to 30 MPa and a treatment time within the range from 2 to 48 h, and
(d) subsequently continuing the hydrogenation of the compound containing at least one unsaturated carbon-nitrogen bond.

2. A process as claimed in claim 1, wherein the compound containing at least one unsaturated carbon-nitrogen bond is a C₄-C₈-alkylnitrile, a C₅-C₈-cycloalkylnitrile, a C₄-C₈-alkyldinitrile or a C₅-C₈-cycloalkyldinitrile.

3. A process as claimed in claim 1 or 2, wherein adiponitrile is used to obtain 6-aminocapronitrile and hexamethylenediamine.

4. A process as claimed in any of claims 1 to 3, wherein the hydrogenation of the compound containing at least one unsaturated carbon-nitrogen bond is carried out in suspension at a temperature within the range from 40 to 150°C and at a pressure within the range from 2 to 20 MPa.

5. A process as claimed in any of claims 1 to 3, wherein the hydrogenation of the compound containing at least one unsaturated carbon-nitrogen bond is carried out in a fixed-bed reactor in a downflow or upflow process at a temperature within the range from 30 to 200°C and at a pressure within the range from 2 to 30 MPa.

6. A process for regenerating a cobalt- and/or iron-containing catalyst, which comprises treating the catalyst with hydrogen at from 150 to 400°C using a hydrogen pressure within the range from 0.1 to 30 MPa and a treatment time within the range from 2 to 48 h.

## Revendications

1. Procédé de préparation de composés contenant des groupes NH₂ par hydrogénation de composés, qui contiennent au moins une liaison carbone-azote insaturée, par de l'hydrogène en présence d'un catalyseur, à des températures non inférieures à la température ambiante et à une pression partielle d'hydrogène élevée, éventuellement en présence d'un solvant, caractérisé en ce que
(a) on met en oeuvre, comme catalyseur, un catalyseur contenant du cobalt et/ou du fer, et
(b) après diminution de la transformation, par rapport au composé à hydrogéner, et/ou de la sélectivité, par rapport au produit souhaité, en dessous d'une valeur définie ou après augmentation de la quantité d'un produit secondaire non souhaité au-dessus d'une valeur définie, on interrompt l'hydrogénation, en stoppant l'alimentation des composés à hydrogéner ainsi que du solvant éventuellement présent,
(c) on traite le catalyseur avec de l'hydrogène à une température de l'ordre de 150 à 400°C, en sélectionnant la pression d'hydrogène dans la gamme de 0,1 à 30 MPa et le temps de traitement dans la gamme de 2 à 48 h, et
(d) on poursuit ensuite l'hydrogénation des composés contenant au moins une liaison carbone-azote insaturée.

2. Procédé suivant la revendication 1, caractérisé en ce que, comme composés contenant au moins une liaison carbone-azote insaturée, on met en oeuvre des C₄-C₈-alkylnitriles, C₅-C₈-cycloalkylnitriles, C₄-C₈-alkyldinitriles ou C₅-C₈-cycloalkyldinitriles.

3. Procédé suivant la revendication 1 ou 2, caractérisé en ce qu'on met en oeuvre de l'adipodinitrile avec obtention de 6-amino-capronitrile et d'hexaméthylènediamine.

4. Procédé suivant l'une des revendications 1 à 3, caractérisé en ce qu'on effectue l'hydrogénation du composé contenant au moins une liaison carbone-azote insaturée en suspension à une température de l'ordre de 40 à 150°C et à une pression de l'ordre de 2 à 20 MPa.

5. Procédé suivant l'une des revendications 1 à 3, caractérisé en ce qu'on effectue l'hydrogénation du composé contenant au moins une liaison carbone-azote insaturée dans un réacteur à lit fixe en un mode de fonctionnement tourbillonnaire ou en fond de cuve à une température de l'ordre de 30 à 200°C et à une pression de l'ordre de 2 à 30 MPa.

6. Procédé de régénération de catalyseurs contenant du cobalt et/ou du fer, caractérisé en ce qu'on traite les catalyseurs avec de l'hydrogène à une température de l'ordre de 150 à 400°C et à une pression d'hydrogène de l'ordre de 0,1 à 30 MPa et pendant une durée de traitement de l'ordre de 2 à 48 h.
